# EUROPÄISCHE PATENTSCHRIFT

(11) **EP 3 417 899 B1**
(45) Veröffentlichungstag und Bekanntmachung des Hinweises auf die Patenterteilung: **19.08.2020**
(21) Anmeldenummer: 18000528.2
(22) Anmeldetag: 15.06.2018
(51) Int. Cl.: A61M 16/06

(54) **ATEMMASKE-SCHNITTSTELLE MIT GERINGEN BEDIENKRÄFTEN**
RESPIRATORY MASK INTERFACE WITH LOW CONTROL FORCES
INTERFACE DE MASQUE D'ASPIRATION À FAIBLE FORCE D'ACTIONNEMENT

(30) Priorität: 19.06.2017 DE 102017005703; 19.06.2017 DE 102017005704
(43) Veröffentlichungstag der Anmeldung: 26.12.2018
(73) Patentinhaber: Löwenstein Medical Technology S.A., 2557 Luxembourg (LU)
(72) Erfinder: Frerichs, Arnold, 21614 Buxtehude (DE); Bechtel, Martin, 21423 Winsen / Luhe (DE); Eifler, Martin, 25348 Glückstadt (DE); Gardein, Joachim, 38430 Icod de los Vinos, Tenerife Islas Canarias (ES)
(74) Vertreter: Marx, Thomas

(56) Entgegenhaltungen:
- WO-A1-2007/143793
- WO-A1-2013/006899
- WO-A1-2016/072868
- WO-A2-03/035156
- DE-A1-102005 031 541
- US-A1- 2013 276 790

## Beschreibung

Bei einer Atemmaske wie beispielsweis einer Beatmungsmaske oder einem beliebigen anderen Beatmungsinterface ist üblicherweise im Maskengrundkörper ein lösbarer und drehbarer Adapter für einen Schlauchanschluss angeordnet. Da der Adapter leicht austauschbar / reinigbar sein muss, ist er - beispielsweise über eine Dreh-Rastverbindung - lösbar. Die Erfindung geht von einem Bedienelement einer Atemmaske mit einer Schnittstelle aus. Das Bedienelement weist ein um eine Drehachse drehbares Griffteil mit einer umfangsseitigen Greiffläche auf. Dabei ist das Griffteil in unterschiedliche Drehpositionen bringbar, wodurch die Schnittstelle bedient wird. Im Bereich der Schnittstelle sind zudem Rastelemente vorhanden. Beispiele für eine solche Schnittstelle zeigen die Atemmaskes der DE 10 2005 041 716 A1 und der DE 10 2005 041 717 A1.

Je nach Ausgestaltung der Bedienelemente müssen teilweise hohe Bedienkräfte übertragen werden können. Dies ist insbesondere bei rein mechanisch ausgebildeten Bedienelementen der Fall, wenn eine zusätzliche Verrastung vorgesehen ist. Wenn man drehbetätigte Bedienelemente nur mit einer umfangseitigen Greiffläche versieht, so müssen die Bedienelemente im Durchmesser vergleichsweise mächtig ausgebildet sein, um die hohen Bedienkräfte ergonomisch vertretbar und sicher übertragen zu können.

Das Dokument US 2013/0276 790 A1 offenbart eine dreidimensionale Bewegung eines Headgear Connectors, um Kopfbewegungen eines Trägers, zu mildern.

Die Atemmaske der DE 10 2005 041 716 A1 und der DE 10 2005 041 717 A1 sind aus einer Vielzahl von Bauteilen konstruiert. Dieses bedingt eine recht massive und schwere Ausgestaltung.

Folgende Dokumente offenbaren auch Atemmasken, US 2013/276790, DE 10 2005 031541, WO 03/035156, WO 2013/006899, WO 2016/072868 und WO 2007/143793.

Der vorliegenden Erfindung liegt die Aufgabe zu Grunde, 'ein Bedienelement der gattungsgemäßen Art bereitzustellen, mit dem hohe Bedienkräfte bei einer trotzdem zierlichen Ausgestaltung übertragen werden können.

Diese Aufgabe wird mit den Merkmalen von Patentanspruch 1 gelöst. Vorteilhafte Ausführungen beziehungsweise Weiterbildungen der Erfindung sind den Unteransprüchen oder der Beschreibung entnehmbar.

Erfindungsgemäß wird nun vorgeschlagen, dass statt der umfangsseitigen Greiffläche der DE 10 2005 041 716 A1 und der DE 10 2005 041 717 A1 dem Sicherungsring ein sich radial nach außen erstreckendes Griffteil angeformt ist, welches dazu ausgebildet und geeignet ist, um als Andruckfläche oder Greifmittel für Finger oder die Hand eines Bedieners zu dienen.

Die Erfindung betrifft daher ein Atemmaske mit einer Schnittstelle für die Verbindung von zumindest zwei Bauteilen, wobei die Schnittstelle rund oder oval ausgeführt ist und einen maximalen Durchmesser (D) aufweist und die Bauteile jeweils zumindest einen Teilbereich der Schnittstelle bereitstellen dadurch gekennzeichnet, dass das Bauteil ein Griffteil aufweist, welches sich von dem Teilbereich radial nach außen erstreckt und welches als Andruckfläche oder Greifmittel ausgebildet ist, die/das für die Bedienung mit dem Finger oder der Hand eines Bedieners (B) geeignet ist,, wobei das Bauteil zwei Griffteile aufweist, welche sich von dem Teilbereich radial nach außen erstrecken und welche als Andruckfläche oder Greifmittel ausgebildet sind, die/das für die Bedienung mit dem Finger oder der Hand eines Bedieners (B) geeignet sind , wobei die Teilbereiche in einem Montagezustand formschlüssig ineinander greifen und um eine Drehachse bewegbar sind, wozu das Griffteil in unterschiedliche Drehpositionen relativ zu den zwei Griffteilen drehbar ist.

Die Griffteile sollten also etwa so bemessen sein, dass Finger eines Bedieners ausreichend Platz auf diesen finden.

Auf diese Weise ist es für den Bediener leicht möglich, sowohl Radialkräfte als auch Axialkräfte auf die Schnittstelle übertragen zu können. Insgesamt ist somit die Übertragung hoher Bedienkräfte möglich, wobei der Durchmesser der umfangsseitigen Greiffläche nicht vergrößert werden muss. Dies ist einem ansprechenden, filigranen Design für die gesamte Atemmaske sehr zuträglich.

Die Erfindung ist auch dadurch gekennzeichnet, dass die Finger eines Bedieners ausreichend Platz auf diesen finden.

Die Erfindung ist auch dadurch gekennzeichnet, dass ein Griffteil als Teil der Stirnstütze und einteilig mit der Stirnstütze ausgebildet, wobei die Stirnstütze zudem Kontaktstellen für die Bänderung bereitstellt.

Die Erfindung ist auch dadurch gekennzeichnet, dass zwei Griffteile als Teil des Körpers und einteilig mit dem Körper ausgebildet, wobei die Griffteile zudem Kontaktstellen für die Bänderung bereitstellen.

Die Erfindung ist auch ergänzend dadurch gekennzeichnet, dass die Oberfläche der Griffteile mit reibungserhöhenden Mitteln (Noppung oder Riffelung) versehen ist, um die Haptik zu verbessern.

Die Erfindung ist bevorzugt dadurch gekennzeichnet, dass die Schnittstelle der Verbindung von drei Bauteilen dient, wobei die Bauteile Maskenkörper, Schlauchstutzen und Stirnstütze sind.

Die Erfindung ist auch dadurch gekennzeichnet, dass das Griffteil länger als Durchmesser (D) der Schnittstelle ist.

Das Griffteil ist der Träger der Stirnabstützung.

Der Durchmesser (D) der Schnittstelle ist der maximale Durchmesser (D) der Aussparung im Bauteil (3).

Die Erfindung ist alternativ auch dadurch gekennzeichnet, dass zwei Griffteile jeweils 50% - 200 % der Abmessung des Durchmessers (D) der Schnittstelle lang sind.

Die Erfindung ist ergänzend auch dadurch gekennzeichnet, dass zwei Griffteile symmetrisch an dem Bauteil einander gegenüber angeordnet sind.

Die zwei Griffteile (7, 9) können auch abgewinkelt zum Bauteil (3) angeordnet sein.

Die Erfindung ist auch dadurch gekennzeichnet, dass im Bereich der Schnittstelle Einführschrägen vorgesehen sind.

Die Erfindung ist bevorzugt auch dadurch gekennzeichnet, dass im Bereich der Schnittstelle in den Teilbereichen Rastmittel vorgesehen sind.

Die Erfindung ist ergänzend auch dadurch gekennzeichnet, dass im Bereich der Schnittstelle in den Teilbereichen zumindest zwei Rastmittel vorgesehen sind die symmetrisch angeordnet sind.

Die Erfindung ist alternativ auch dadurch gekennzeichnet, dass die Einführschrägen und die Rastmittel so ausgestaltet und angeordnet sind, dass die Bedienkräfte, die zum Lösen der Verrastung notwendig sind, höher sind als die Bedienkräfte, die zum Schließen der Verrastung notwendig sind.

Die Erfindung ist auch dadurch gekennzeichnet, dass die Bauteile zumindest einen Zeiger (Z) und zumindest ein Symbol (S) aufweisen, wobei durch Drehung des Griffteils zumindest ein Zeiger (Z) auf zumindest ein Symbol (S) ausrichtbar ist, wobei das Symbol (S) ortsfest ist und der Zeiger (Z) relativ zum Symbol (s) drehbar ausgebildet ist.

Die Erfindung ist nicht auf das bzw. die obigen Ausführungsbeispiele beschränkt. Diese wurden nur zur allgemeinen Erläuterung des Kerngedankens der Erfindung herangezogen. Die Erfindung kann im Rahmen ihres Schutzumfangs vielmehr auch andere als die zuvor beschriebenen Ausführungsbeispiele bzw. Ausprägungen annehmen. Hierbei kann sie insbesondere auch solche Merkmale aufweisen, die eine Kombination aus Einzelmerkmalen der jeweiligen Ansprüche darstellen.

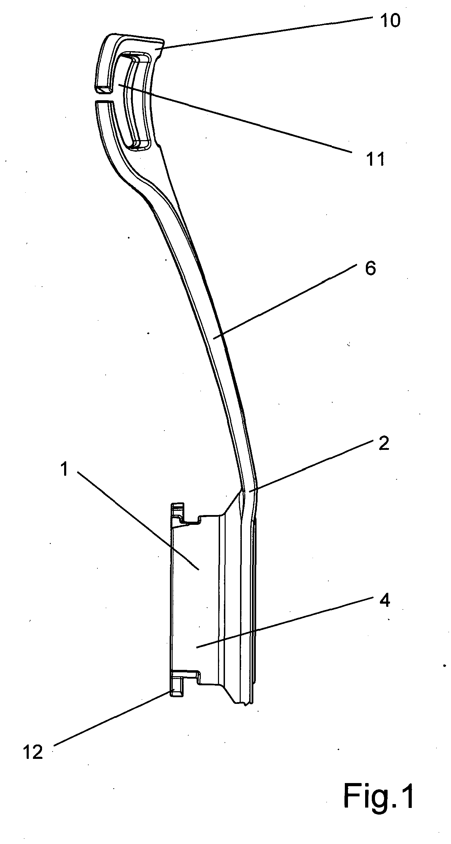

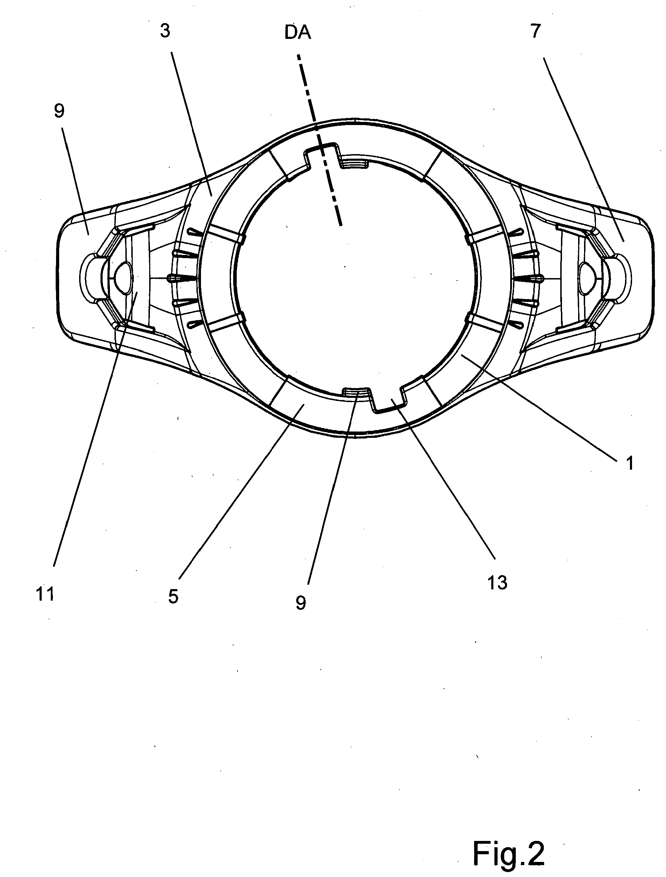

Figur 1 zeigt das Atemmaske mit einer Schnittstelle (1) für die Verbindung von zumindest zwei Bauteilen (2, 3). Die Schnittstelle (1) ist beispielsweise rund oder oval ausgeführt und weist einen maximalen Durchmesser (D) auf. Die Bauteile (2, 3) stellen jeweils zumindest einen Teilbereich (4, 5) der Schnittstelle bereit. Das Bauteil (2) weist ein Griffteil (6) auf, welches sich von dem Teilbereich (4) radial nach außen erstreckt und welches als Andruckfläche oder Greifmittel für Finger oder die Hand eines Bedieners (B) ausgebildet ist. Das Bauteil (3) weist zwei Griffteile (7, 9) auf, welche sich von dem Teilbereich (5) radial nach außen erstrecken und welche als Andruckfläche oder Greifmittel für Finger oder die Hand eines Bedieners (B) ausgebildet sind. Die Teilbereiche (4, 5) greifen in einem Montagezustand formschlüssig ineinander und sind um eine Drehachse (DA) bewegbar. Dazu ist das Griffteil (6) in unterschiedliche Drehpositionen relativ zu den zwei Griffteilen (7, 9) drehbar. Zu erkennen ist die Rotationssymmetrie um eine im Mittelpunkt des inneren Radius befindliche, senkrecht aus der Zeichenebene herausragende Achse (DA) um 180°. Die Symmetrie bedingt, dass die Teile in beliebiger Orientierung montiert werden können. Durch Codierung der Bajonett-Position, z.B. bei drei Bajonetten, ist auch eine asymmetrische Auslegung möglich.

Die Griffteile (6,7,9) sind so bemessen, dass die Finger eines Bedieners ausreichend Platz auf diesen finden.

Das Griffteil (6) ist als Teil der Stirnstütze (10) und einteilig mit der Stirnstütze (10) ausgebildet, wobei die Stirnstütze zudem Kontaktstellen (11) für eine nicht dargestellte Bänderung bereitstellt.

Die zwei Griffteile (7,9) sind als Teil des Körpers und einteilig mit dem Körper (3) ausgebildet. Die Griffteile weisen zudem Kontaktstellen (11) für eine Bänderung auf.

Die Schnittstelle (1) dient der Verbindung von drei Bauteilen (2, 3), wobei die Bauteile Maskenkörper (3), Schlauchstutzen (nicht dargestellt) und Stirnstütze (2) sind. Der Schlauchstutzen weist z.B. einen kugelgelenkförmigen Bereich auf, der von der Stirnstütze (2) zumindest teilweise umgeben und gehalten wird. Bevorzugt wird der kugelgelenkförmige Bereich vom Teilbereich (4) aufgenommen.

Das Griffteil (6) ist länger als Durchmesser (D) der Schnittstelle.

Die zwei Griffteile (7, 9) sind ähnlich lang (50% - 200 %) wie die Abmessung des Durchmessers (D) der Schnittstelle.

Die zwei Griffteile (7, 9) sind hier symmetrisch an dem Bauteil (3) einander gegenüber angeordnet sind.

In dem Bereich (4,5) der Schnittstelle sind Einführschrägen (13) und Rastmittel (12) vorgesehen, die hier symmetrisch in dem Bereich (4,5) angeordnet sind. Die Bauteile 2,3) können daher in beliebiger Orientierung zueinander montiert werden.

Die Einführschrägen und die Rastmittel (12,13) sind so ausgestaltet und angeordnet, dass die Bedienkräfte, die zum Lösen der Verrastung notwendig sind, höher sind als die Bedienkräfte, die zum Schließen der Verrastung notwendig sind. Dies ermöglicht eine Sicherung der Verbindung der Bauteile (2,3) .

Das Bajonett an der Stirnstütze ist radial nach außen weisend realisiert, korrespondierende Aufnahme sind am inneren Radius im Maskenkörper vorgesehen. Das Bajonett am Sicherungsring rastet in Fügerichtung in den Maskenkörper ein, dies Erleichtert die Findung und Führung.

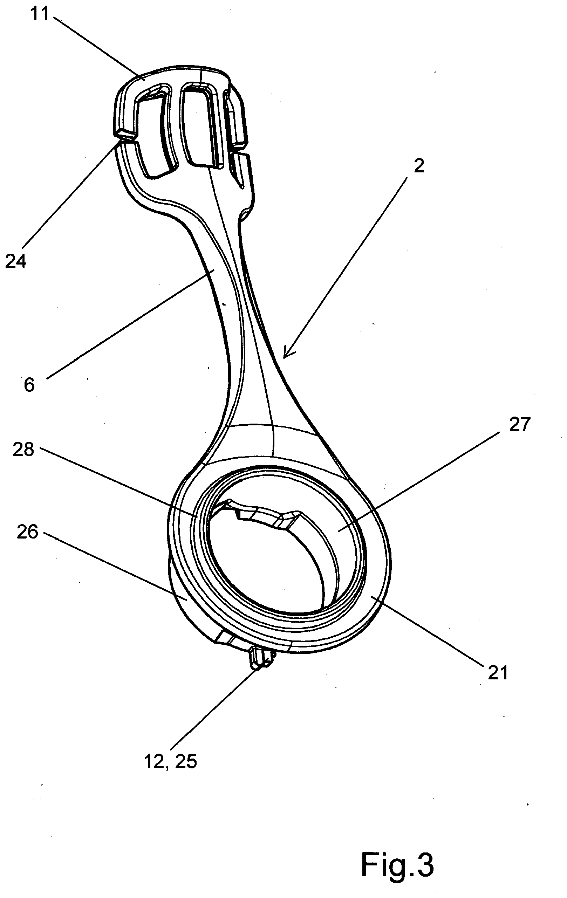

Fig. 3 zeigt eine erfindungsgemäße Ausführungsform einer Stirnstütze (2). Die Basis der Stirnstütze (2) bildet der Stirnstützenverbinder (21), mit dem die Stirnstütze (2) am Maskenkörper (3) befestigt wird. Ein Steg (6) verbindet den Stirnstützenverbinder (21) mit der Bandankopplung (11) am Kopf der Stirnstütze (2). Die Bandankopplung (11) besteht aus zumindest zwei, bevorzugt drei, alternativ vier horizontal nebeneinander angeordneten, in vertikaler Richtung länglich ausgedehnten Aussparungen in der Struktur der Stirnstütze (2). Die beiden äußeren Aussparungen weisen jeweils einen zusätzlichen Spalt (24) in der nach außen gewandten Struktur der Stirnstütze (2) auf. Diese Spalte (24) können zum Einfädeln der Haltebänder auf Stirnhöhe eines Patienten in die Bandankopplung (11) genutzt werden. Alternativ kann das Band auch durch die mittig positionierte Aussparung geführt werden.

Es ist auch an andere Ausführungsformen der Bandankopplung (11) gedacht. Beispielsweise kann diese auch nur durch zwei -Aussparungen mit zusätzlichem Spalt (24) umgesetzt werden.

Der Steg (6) ist in seiner Breite in der gezeigten Ausführungsform gegenüber dem Kopfteil mit Bandankopplung (11) und dem Stirnstützenverbinder (21) signifikant reduziert. Dies ermöglicht ein ansprechendes, schlankes Design und die Einsparung von Material.

Der Stirnstützenverbinder (21) bietet zur Vervollständigung des im Maskenkörper (3) angelegten Bajonettverschlusses eine in Positionierung und Anzahl der im Maskenkörper vorhandenen Kanäle (8, 13) entsprechende Anzahl an Verschlusszähnen (12, 25). Diese stehen auf der äußeren Kontur des Verbindungsringes (4,26) radial nach außen und weisen ihrerseits jeweils einen weiteren radial nach außen gerichteten Zahn auf, der gemeinsam mit dem Rastzahn (14) im Maskenkörper (3) für die Arretierung von Maskenkörper (3) und Stirnstütze (2) in der vorgesehenen Position sorgt. Die innere Kontur des Stirnstützenverbinders (21) ist als Kugelkäfig (27) ausgeführt, der das Anschlussstück (33) beweglich lagern kann. Zum Anschlussstück (33) hin wird der Kugelkäfig (27) durch eine sich nach außen verengende Ringstruktur (28) begrenzt.

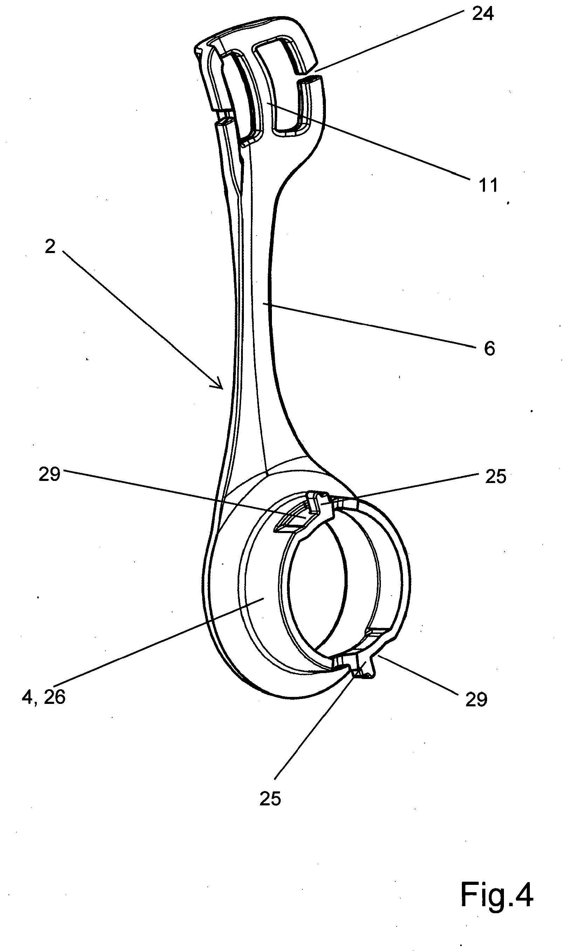

Fig. 4 zeigt eine perspektivische Darstellung einer Stirnstütze (2) von hinten. Die dargestellte Stirnstütze (2) weist zwei Verschlusszähne (25) auf. Jedoch sind auch mehr Verschlusszähne (25) passend zum jeweiligen Maskenkörper (3) denkbar. Erkennbar sind Aussparungen (29) in der äußeren Kontur des Verbindungsringes (26) neben den Verschlusszähnen (25). Diese Aussparungen (29) dienen als zusätzliche Ausströmkanäle und leiten die ausgeatmeten Gase um den jeweiligen Verschlusszahn (25) zum entsprechenden im Maskenkörper (3) angeordneten Kanal (8). Zusätzlich ist auch die Nutzung einer solchen Aussparung (29) als mechanische Kodierung für eine definierte Ausrichtung von Maskenkörper (3), Stirnstütze (2) und Maskenwulst (3) denkbar. Dafür muss die Struktur des Maskenkörpers (3) im Bereich seiner mechanischen Kodierung (15) entsprechend durchlässig sein.

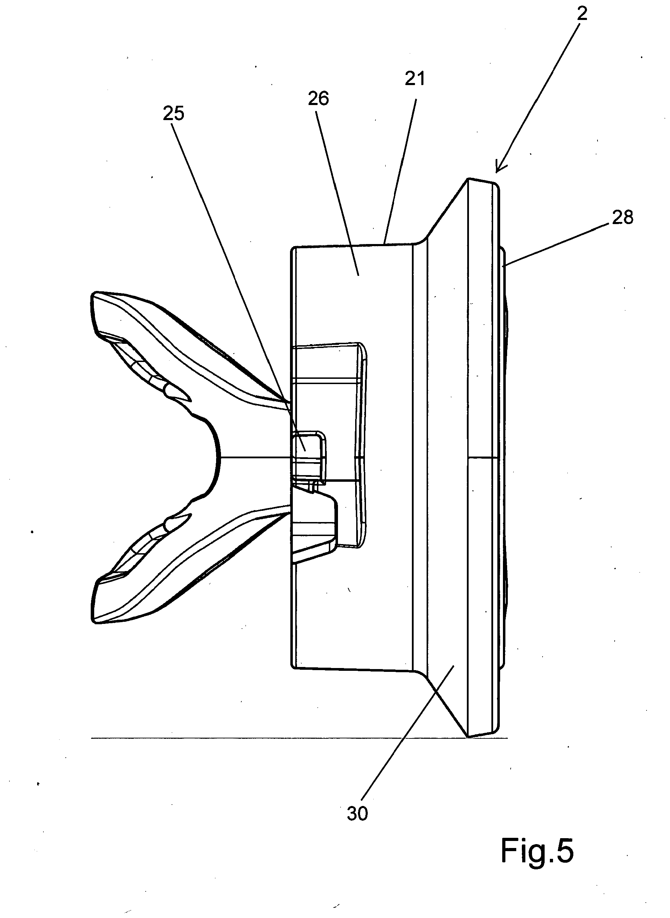

In Fig. 5 ist die Stirnstütze (2) aus Fig. 15 von unten dargestellt. Zu erkennen ist die der inneren Kontur des Maskenkörpers (3) angepasste äußere Kontur des Stirnstützenverbinders (21), die sich ebenfalls aus einem Ring und einem Trichter zusammensetzt. Diese Kontur bildet die stirnstützenseitige Abströmoberfläche (30) und definiert gemeinsam mit der innenliegenden Oberfläche des Maskenkörpers (3) die Form der Abströmkanäle und der Ausatemspalte.

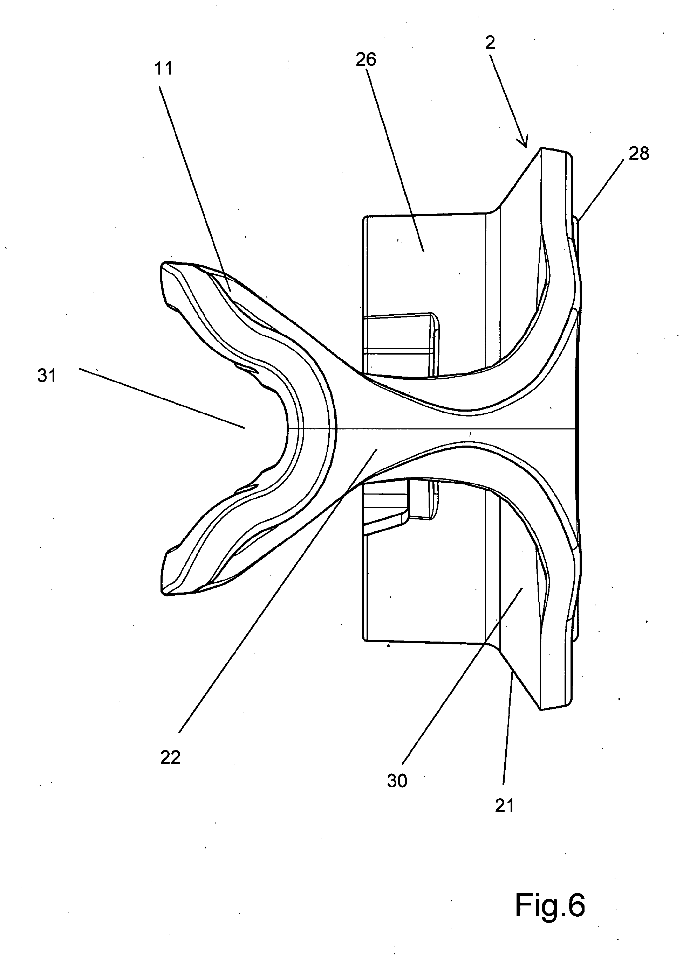

Fig. 6 zeigt die Stirnstütze (2) von oben. Zu erkennen ist, dass das Kopfteil der Stirnstütze (2) in Relation zum Stirnstützenverbinder (21) zu einem Patienten hin ragt. Die Form des die Bandankopplung (11) beherbergenden Kopfes der Stirnstütze (2) weist eine Aussparung (31) auf, die zwischen der Stirn eines Patienten und der Bandankopplung (23) in der Stirnstütze (2) einen Raum bietet.

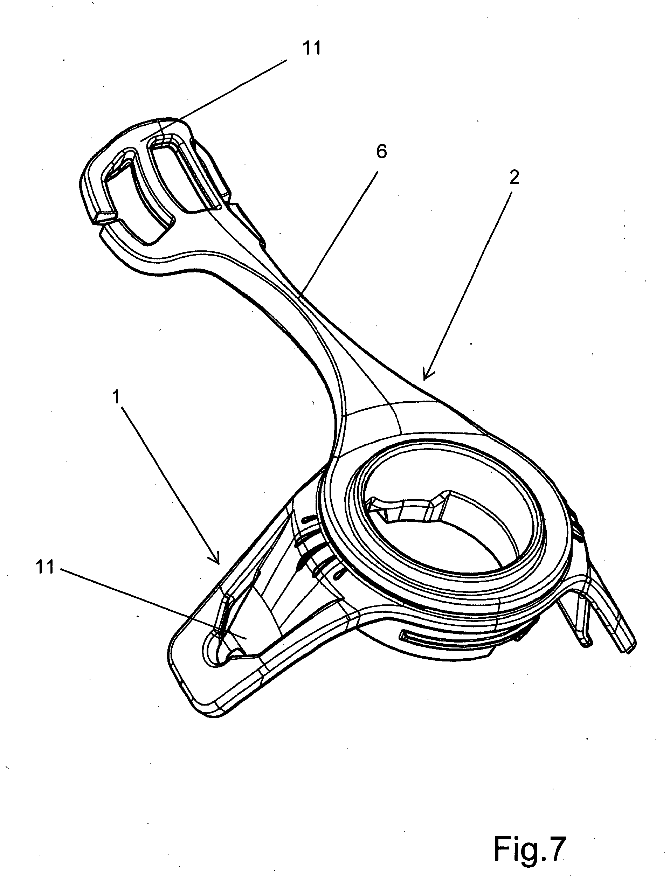

In Fig. 7 sind Maskenkörper (3) und Stirnstütze (2) als montiertes Modul in perspektivischer Darstellung zu sehen. Bei einer rotationssymmetrischen Ausführung des Maskenkörpers (3) kann dieser in genau zwei um 180° verschobenen Positionen mit der Stirnstütze (2) verbunden werden, die funktional identisch sind. Damit lässt sich eine Vereinfachung der Montage der Module der Atemmaske erreichen. Maskenkörper (3) und Stirnstütze (2) können unter Verwendung von Distanzelementen (40,41), welche in der Schnittstelle angeordnet sind, konnektiert werden. Alternativ kann die mechanische Kopplung auch ohne Verwendung von Distanzelementen (40,41) erfolgen.

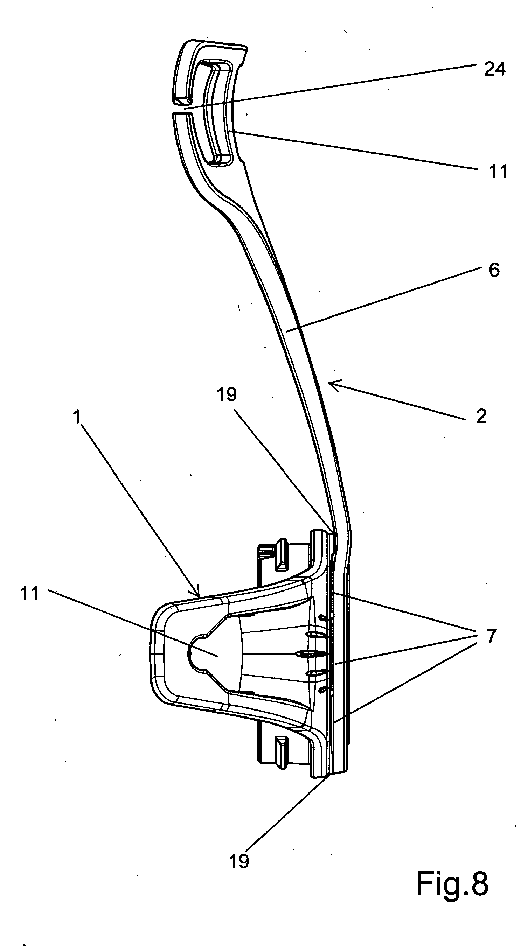

Fig. 8 zeigt eine seitliche Ansicht des montierten Moduls aus Maskenkörper (3) und Stirnstütze (2). Die zwischen den Abströmoberflächen des Maskenkörpers (3), sowie den Ausströmkanälen und der Abströmoberfläche (30) des Stirnstützenverbinders (21) verbleibenden Spalte (17), dienen als Leitstruktur für die ausgeatmete Luft. Durch die erhöhte Struktur im Bereich der Kanäle (19) im Maskenkörper (3) wird an der oberen Seite in Richtung der Stirnstütze (2) eine Abdichtung des Ausatemspaltes in einem definierten Bereich erreicht. Dies dient der Vermeidung der Ausströmung von Luft in für einen Patienten unangenehme Bereiche, insbesondere die Augen.

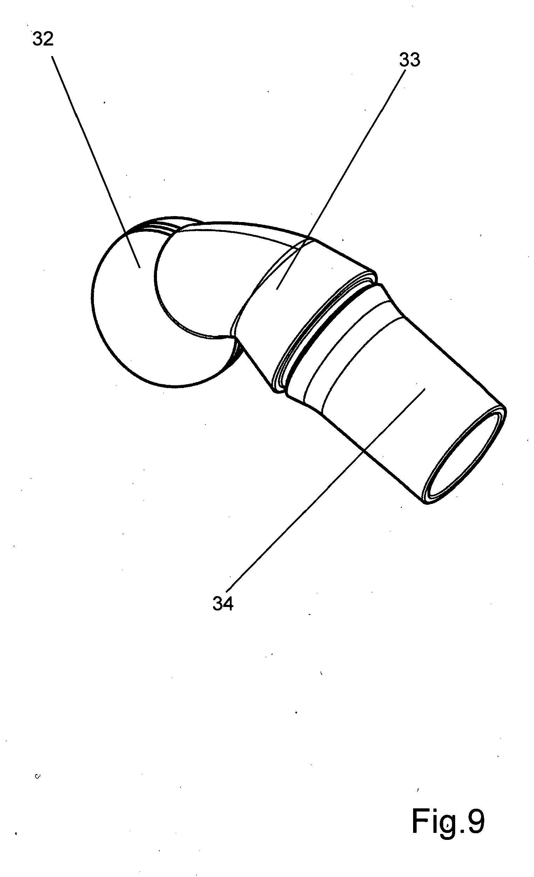

Fig. 9 zeigt eine perspektivische Darstellung eines erfindungsgemäßen Anschlussstückes (33). Das Anschlussstück ist als abgewinkelte Rohrstruktur ausgeführt und weist an einem Ende eine Oberflächenkontur (32) auf, die einer Teilkugel entspricht. Diese Teilkugel (32) dient der beweglichen Verbindung von Anschlussstück und Stirnstütze (2) mittels des im Stirnstützenverbinder (21) realisierten Kugelkäfigs (27).

Am anderen -Ende ist eine drehbar gelagerte Hülse (34) als Anschluss für einen nicht dargestellten Schlauch montiert.

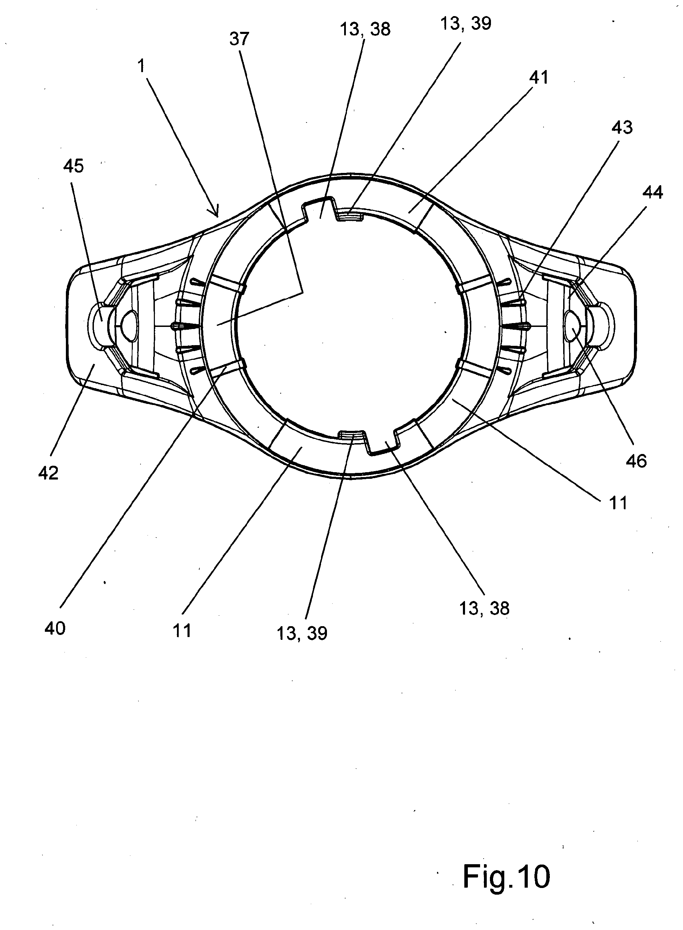

Fig. 10 zeigt den Maskenkörper (3) in frontaler Ansicht. Zu erkennen ist die Rotationssymmetrie um eine im Mittelpunkt des inneren Radius befindlichen, senkrecht aus der Zeichenebene herausragenden Achse um 180°. Ersichtlich sind die radial aus der inneren Kontur (37) herausragenden Distanzrippen (40) und die verstärken Bereiche (41) um die Ausströmkanäle (8,38). Auch ist die Form der Rastzähne für den Bajonettverschluss zu sehen, der die Bajonettverbindung durch die Verrastung mit dem Gegenstück an der Stirnstütze (2) gegen ungewolltes Verdrehen und Lösen sichert.
Zu erkennen sind mehrere Ausströmflächen (37) auf der innenliegenden Oberfläche des Maskenkörpers (3). Die innenliegende Oberfläche des Maskenkörpers (3) öffnet sich nach vorn trichterförmig, während ihre Grundform im hinteren, einem Patienten zugewandten Bereich durch ein Rohr konstanten Durchmessers definiert wird. In der inneren Kontur des Maskenkörpers (3) befinden sich mindestens zwei Kanäle (8,38), die als Aussparung im rohrförmigen Bereich realisiert sind und die sich bis in den trichterförmigen Bereich erstrecken. Diese Kanäle (8,38) fungieren in doppelter Funktion sowohl als Aufnahmen für die mechanische Ankopplung der Stirnstütze (2), die beispielsweise als Bajonettverschluss realisiert werden kann, als auch als Abströmkanäle für ausgeatmete Atemgase, deren vollständige Kontur gemeinsam von Maskenkörper (3) und Stirnstütze (2) definiert wird. Für den Bajonettverschluss ist im hinteren Bereich neben jedem Kanal (8,38) jeweils ein Zahn (39) vorgesehen, der radial nach innen aus der inneren Kontur des Maskenkörpers (3) herausragt und der den benötigten mechanischen Widerstand für den Verschluss bietet. Des Weiteren sind auf der inneren Oberfläche des Maskenkörpers (3) mehrere Distanzrippen (40) angeordnet, die eine Verbindung von Maskenkörper (3) und Stirnstütze (2) ohne Spiel bzw. unter Vorspannung ermöglichen. Im Bereich (41) der Abströmkanäle (8, 38) ist das Material im Ring- und Trichterbereich entsprechend der Höhe der Distanzrippen (40) verstärkt. Die Distanzrippen (40) und die verstärkten Bereiche (41) begrenzen zudem seitlich die Ausströmflächen (37).
Denkbar sind auch zusätzliche Aussparungen im trichterförmigen Bereich der inneren Oberfläche des Maskenkörpers (3), um einen oder mehrere zusätzliche Ausströmkanäle zu formen.

Innerhalb der inneren Kontur des Maskenkörpers (3) ist auch eine ringförmig umlaufende Vertiefung denkbar, die die ausströmenden Atemgase gleichmäßig auf die angelegten Ausströmflächen (7) verteilt. Diese könnte z.B. im Übergangsbereich von Rohr- zu Trichterform der innenliegenden Oberfläche des Maskenkörpers (3) realisiert werden.

An seinen Seiten weist der Maskenkörper (3) zwei Flügel (7,9,42) auf, die jeweils eine mechanische Struktur zur Aufnahme einer Bandankopplung (11) aufweisen. Die Bandankopplung kann beispielsweise durch einen Clip (nicht dargestellt) erfolgen. Im vorderen Bereich des Maskenkörpers (3) ist dieser auf Höhe der Flügel (42) mit Noppen (43) versehen, die eine bessere Griffigkeit bei der Handhabung der Atemmaske ermöglichen. Die Noppen können auch als Strahlen oder Pfeile ausgeführt sein, die die Richtung der abströmenden Atemgase anzeigen (43).

Der Kanal (38) und wird in seiner Grundform hinter dem Zahn (39) zu einer L-förmigen Aussparung ergänzt, was die typische Steck- und Schraubverbindung ermöglicht. Im Konturbereich hinter dem Zahn (39) ist ein Rastzahn vorgesehen, der die Verbindung sichert.
Zusätzlich kann in einer Ausführungsform der Atemmaske eine mechanische Kodierung im Maskenkörper (3) derart realisiert, dass der Maskenwulst sich nur in vorgesehenen Positionen mit dem Maskenkörper (3) verbinden lässt. Diese Verbindung erfolgt durch ein Aufstecken des Maskenwulstes auf die äußere Kontur (16) des Maskenkörpers (3). Gesichert wird die Verbindung in der gezeigten Ausführungsform durch mindestens einen Hinterschnitt auf der äußeren Kontur des Maskenkörpers (3). Denkbar sind neben der dargestellten kreisrunden äußeren Kontur auch andersförmige Ausführungen. Insbesondere ist an eine ovale Form der äußeren Kontur gedacht. In Kombination mit der Form der Verbindungsöffnung des Maskenwulstes, die mit einem inneren Umfang kleiner oder gleich der äußeren Kontur des Maskenkörpers (3), auch anders geformt sein kann, können die Steifigkeit und das Verhältnis von Höhe und Breite des Maskenwulstes eingestellt werden. Insbesondere ist an eine Kombination von ovaler Ausführung der äußeren Kontur und einer kreisrunden Ausführung der Verbindungsöffnung des Maskenwulstes gedacht. Neben der gezeigten Verbindung von Maskenwulst und Maskenkörper (3) durch einen Hinterschnitt, sind auch andere Optionen denkbar. Zum Beispiel ist auch ein Verkleben der Module mit Hilfe eines Zweikomponentenklebstoffs möglich. Die von im Bereich der Flügel (42) bereitgestellte mechanische Aufnahmestruktur für die Bandankopplung besteht im Wesentlichen aus einer Federplatte (44) in Kombination mit einer Aussparung in der Struktur des Flügels (42). Der Clip wird durch eine Sicherung (46) in der Aufnahmestruktur gehalten. Die Sicherung weist bevorzugt einen Radius auf.

## Patentansprüche

1. Atemmaske mit einer Schnittstelle (1) für die Verbindung von zumindest zwei Bauteilen (2, 3), einem Bauteil Stirnstütze (2), und einem Bauteil Maskenkörper (3), wobei die Schnittstelle (1) rund oder oval ausgeführt ist und einen maximalen Durchmesser (D) aufweist und die Bauteile (2, 3) jeweils zumindest einen Teilbereich (4, 5) der Schnittstelle bereitstellen, wobei das Bauteil (2) ein Griffteil (6) aufweist, das als Teil der Stirnstütze (10) ausgebildet ist, und welches sich von dem Teilbereich (4) : und von der Drehachse (DA) gesehen radial nach außen erstreckt und welches als Andruckfläche oder Greifmittel ausgebildet ist, die/das für die Bedienung mit dem Finger oder der Hand eines Bedieners (B) geeignet ist, wobei das Bauteil (3) zwei Griffteile (7, 9) aufweist, welche sich von dem Teilbereich (5) radial nach außen erstrecken und welche als Andruckfläche oder Greifmittel ausgebildet sind, die/das für die Bedienung mit dem Finger oder der Hand eines Bedieners (B) geeignet sind und die zudem Kontaktstellen (11) für eine Bänderung aufweisen, wobei die Teilbereiche (4, 5) in einem Montagevorgang um eine Drehachse (DA) bewegbar sind und dadurch formschlüssig ineinander greifen, wozu das Griffteil (6) in unterschiedliche Drehpositionen relativ zu den zwei Griffteilen (7, 9) drehbar ist.

2. Atemmaske nach Anspruch 1 **dadurch gekennzeichnet, dass** die Griffteile (6,7,9) so bemessen sind, dass Finger eines Bedieners ausreichend Platz auf diesen finden.

3. Atemmaske nach Anspruch 1 oder 2 **dadurch gekennzeichnet, dass** ein Griffteil (6) als Teil der Stirnstütze (2) und einteilig mit der Stirnstütze (10) ausgebildet ist, wobei die Stirnstütze zudem Kontaktstellen (11) für die Bänderung bereitstellt.

4. Atemmaske nach zumindest einem der vorhergehenden Ansprüche **dadurch gekennzeichnet, dass** zwei Griffteile (7,9) als Teil des Körpers und einteilig mit dem Körper (3) ausgebildet, wobei die Griffteile zudem Kontaktstellen (11) für die Bänderung bereitstellen.

5. Atemmaske nach zumindest einem der vorhergehenden Ansprüche **dadurch gekennzeichnet, dass** die Oberfläche der Griffteile mit reibungserhöhenden Mitteln (beispielsweise Noppung oder Riffelung) versehen ist, um die Haptik zu verbessern.

6. Atemmaske nach zumindest einem der vorhergehenden Ansprüche **dadurch gekennzeichnet, dass** Schnittstelle (1) der Verbindung von drei Bauteilen dient, wobei die Bauteile Maskenkörper (3), Schlauchstutzen und Stirnstütze (2) sind.

7. Atemmaske nach zumindest einem der vorhergehenden Ansprüche **dadurch gekennzeichnet, dass** das Griffteil (6)' länger als der Durchmesser (D) der Schnittstelle ist.

8. Atemmaske nach zumindest einem der vorhergehenden Ansprüche **dadurch gekennzeichnet, dass** zwei Griffteile (7, 9) jeweils 50% - 200 % der Abmessung des Durchmessers (D) der Schnittstelle lang sind.

9. Atemmaske nach zumindest einem der vorhergehenden Ansprüche **dadurch gekennzeichnet, dass** zwei Griffteile (7, 9) symmetrisch an dem Bauteil (3) einander gegenüber angeordnet sind.

10. Atemmaske nach zumindest einem der vorhergehenden Ansprüche **dadurch gekennzeichnet, dass** im Bereich der Schnittstelle Einführschrägen vorgesehen sind.

11. Atemmaske nach zumindest einem der vorhergehenden Ansprüche **dadurch gekennzeichnet, dass** im Bereich der Schnittstelle in den Teilbereichen (4, 5) Rastmittel vorgesehen sind.

12. Atemmaske nach zumindest einem der vorhergehenden Ansprüche **dadurch gekennzeichnet, dass** im Bereich der Schnittstelle in den Teilbereichen (4, 5) zumindest zwei Rastmittel (12,13) vorgesehen sind die symmetrisch angeordnet sind.

13. Atemmaske nach zumindest einem der vorhergehenden Ansprüche **dadurch gekennzeichnet, dass** die Einführschrägen und die Rastmittel (12,13) so ausgestaltet und angeordnet sind, dass die Bedienkräfte, die zum Lösen der Verrastung notwendig sind, höher sind als die Bedienkräfte, die zum Schließen der Verrastung notwendig sind.

14. Atemmaske nach zumindest einem der vorhergehenden Ansprüche **dadurch gekennzeichnet, dass** in der Schnittstelle zwischen Maskenkörper (3) und Stirnstütze (2) sind Distanzelementen (40,41) angeordnet.

15. Atemmaske nach zumindest einem der vorhergehenden Ansprüche **dadurch gekennzeichnet, dass** die Bauteile (2, 3) zumindest einen Zeiger (Z) und zumindest ein Symbol (S) aufweisen, wobei durch Drehung des Griffteils (6,7,9) zumindest ein Zeiger (Z) auf zumindest ein Symbol (S) ausrichtbar ist, wobei das Symbol (S) ortsfest ist und der. Zeiger (Z) relativ zum Symbol (s) drehbar ausgebildet ist.

## Claims

1. A breathing mask with an interface (1) for connecting at least two components (2, 3), a forehead support (2) component and a mask body (3) component, wherein the interface (1) is designed round or oval and has a maximum diameter (D), and the components (2, 3) each provide at least one section (4, 5) of the interface, wherein the component (2) has a grip part (6) that is designed as a part of the forehead support (10) and which extends radially outward viewed from the section (4) and from the axis of rotation (DA), and which is designed as a pressure surface or gripping means that is suitable for being used by the finger or hand of a user (B), wherein the component (3) has two grip parts (7, 9) which extend radially outward from the section (5) and which are designed as a pressure surface or gripping means that are suitable for being used by the finger or the hand of a user (B) and moreover have contact points (11) for a banding, wherein the sections (4, 5) can be moved in an assembly process about an axis of rotation (DA) and thereby engage in each other in a form fit, wherein the grip part (6) can be rotated in different rotary positions relative to the two grip parts (7, 9).

2. The breathing mask according to claim 1, **characterized in that** the grip parts (6, 7, 9) are dimensioned such that fingers of a user have sufficient room thereupon.

3. The breathing mask according to claim 1 or 2, **characterized in that** a grip part (6) is designed as part of the forehead support (2) and as a single part with the forehead support (10), wherein the forehead support moreover provides contact points (11) for the banding.

4. The breathing mask according to at least one of the preceding claims, **characterized in that** two grip parts (7, 9) are designed as part of the body and as a single part with the body (3), wherein the grip parts moreover provide contact points (11) for the banding.

5. The breathing mask according to at least one of the preceding claims, **characterized in that** the surface of the grip parts is provided with friction-increasing means (such as bumps or corrugation) in order to improve the feel.

6. The breathing mask according to at least one of the preceding claims, **characterized in that** the interface (1) serves to connect three components, wherein the components are the mask body (3), tube connector and forehead support (2).

7. The breathing mask according to at least one of the preceding claims, **characterized in that** the grip part (6) is longer than the diameter (D) of the interface.

8. The breathing mask according to at least one of the preceding claims, **characterized in that** two grip parts (7, 9) are each 50%-200% the length of the dimension of the diameter (D) of the interface.

9. The breathing mask according to at least one of the preceding claims, **characterized in that** two grip parts (7, 9) are arranged symmetrically to each other on the component (3).

10. The breathing mask according to at least one of the preceding claims, **characterized in that** insertion bevels are provided in the region of the interface.

11. The breathing mask according to at least one of the preceding claims, **characterized in that** locking means are provided in the region of the interface in the sections (4, 5).

12. The breathing mask according to at least one of the preceding claims, **characterized in that** at least two locking means (12, 13) which are arranged symmetrically are provided in the region of the interface in the sections (4, 5).

13. The breathing mask according to at least one of the preceding claims, **characterized in that** the insertion bevels and the locking means (12, 13) are designed and arranged so that the operating forces which are necessary to release the lock are greater than the operating forces which are necessary to close the lock.

14. The breathing mask according to at least one of the preceding claims, **characterized in that** spacing elements (40, 41) are arranged in the interface between the mask body (3) and forehead support (2).

15. The breathing mask according to at least one of the preceding claims, **characterized in that** the components (2, 3) have at least one indicator (Z) and at least one symbol (S), wherein by rotating the grip part (6, 7, 9), at least one indicator (Z) can be oriented toward at least one symbol (S), wherein the symbol (S) is stationary, and the indicator (Z) is designed to be rotatable relative to the symbol (s).

## Revendications

1. Masque respiratoire avec une interface (1) pour l'assemblage d'au moins deux composants (2, 3), un composant de support frontal (2) et un composant de corps de masque (3), dans lequel l'interface (1) est réalisée ronde ou ovale et présente un diamètre maximal (D) et les composants (2, 3) fournissent respectivement au moins une région partielle (4, 5) de l'interface, dans lequel le composant (2) présente une partie de préhension (6) réalisée comme une partie du support frontal (10), et laquelle s'étend radialement vers l'extérieur vue depuis la région partielle (4) et depuis l'axe de rotation (DA) et laquelle est réalisée comme une surface d'appui ou comme un moyen de prise, laquelle/lequel est adapté(e) pour être utilisé (e) avec le doigt ou la main d'un utilisateur (B), dans lequel le composant (3) présente deux parties de préhension (7, 9) s'étendant radialement vers l'extérieur à partir de la région partielle (5) et réalisées comme une surface d'appui ou comme un moyen de prise, laquelle/lequel est adapté pour être utilisé (e) avec le doigt ou la main d'un utilisateur (B) et présente en outre des zones de contact (11) pour un agencement de brides, dans lequel les régions partielles (4, 5) sont déplaçables autour d'un axe de rotation (DA) dans une opération de montage et s'engagent ainsi l'une dans l'autre par complémentarité de forme, ce pourquoi la partie de préhension (6) peut être tournée dans différentes positions de rotation par rapport aux deux parties de préhension (7, 9).

2. Masque respiratoire selon la revendication 1, **caractérisé en ce que** les parties de préhension (6, 7, 9) sont dimensionnées de manière à fournir suffisamment de place pour les doigts d'un utilisateur.

3. Masque respiratoire selon la revendication 1 ou 2, **caractérisé en ce qu'**une partie de préhension (6) est réalisée comme une partie du support frontal (2) et d'un seul tenant avec le support frontal (10), le support frontal fournissant en outre des zones de contact (11) pour l'agencement de brides.

4. Masque respiratoire selon l'une au moins des revendications précédentes, **caractérisé en ce que** deux parties de préhension (7, 9) sont réalisées comme une partie du corps et d'un seul tenant avec le corps (3), les parties de préhension fournissant en outre des zones de contact (11) pour l'agencement de brides.

5. Masque respiratoire selon l'une au moins des revendications précédentes, **caractérisé en ce que** la surface supérieure des parties de préhension est pourvue de moyens augmentant le frottement (par exemple un bossage, ou une cannelure), pour améliorer l'haptique.

6. Masque respiratoire selon l'une au moins des revendications précédentes, **caractérisé en ce que** l'interface (1) sert à assembler trois composants, les composants consistant en un corps de masque (3), un raccord de tuyau et un support frontal (2).

7. Masque respiratoire selon l'une au moins des revendications précédentes, **caractérisé en ce que** la partie de préhension (6) est plus longue que le diamètre (D) de l'interface.

8. Masque respiratoire selon l'une au moins des revendications précédentes, **caractérisé en ce que** deux parties de préhension (7, 9) présentent une longueur correspondant respectivement à 50% - 200% de la dimension du diamètre (D) de l'interface.

9. Masque respiratoire selon l'une au moins des revendications précédentes, **caractérisé en ce que** deux parties de préhension (7, 9) sont disposées symétriquement l'une en face de l'autre sur le composant (3).

10. Masque respiratoire selon l'une au moins des revendications précédentes, **caractérisé en ce que** des chanfreins d'insertion sont prévus dans la région de l'interface.

11. Masque respiratoire selon l'une au moins des revendications précédentes, **caractérisé en ce que** des moyens d'encliquetage sont prévus dans les régions partielles (4, 5) dans la région de l'interface.

12. Masque respiratoire selon l'une au moins des revendications précédentes, **caractérisé en ce qu'**au moins deux moyens d'encliquetage (12, 13) disposés symétriquement sont prévus dans les régions partielles (4, 5) dans la région de l'interface.

13. Masque respiratoire selon l'une au moins des revendications précédentes, **caractérisé en ce que** les chanfreins d'introduction et les moyens d'encliquetage (12, 13) sont réalisés et disposés de telle façon que les forces d'utilisation nécessaires pour défaire l'encliquetage sont supérieures aux forces d'utilisation nécessaires à la fermeture de l'encliquetage.

14. Masque respiratoire selon l'une au moins des revendications précédentes, **caractérisé en ce que** des éléments d'espacement (40, 41) sont disposés entre le corps de masque (3) et le support frontal (2) dans l'interface.

15. Masque respiratoire selon l'une au moins des revendications précédentes, **caractérisé en ce que** les composants (2, 3) présentent au moins un pointeur (Z) et un symbole (S), dans lequel une rotation de la partie de préhension (6, 7, 9) permet d'orienter au moins un pointeur (Z) sur au moins un symbole (S), le symbole (S) étant fixe et le pointeur (Z) étant rotatif par rapport au symbole (s).
